# EUROPÄISCHE PATENTSCHRIFT

(11) **EP 1 353 931 B1**
(45) Veröffentlichungstag und Bekanntmachung des Hinweises auf die Patenterteilung: **09.11.2005**
(21) Anmeldenummer: 02719707.8
(22) Anmeldetag: 19.01.2002
(51) Int. Cl.: C07F 13/00, C07F 15/00, C07B 43/04, C07B 41/02, C07B 37/04, A61K 33/24, A61P 35/00

(54) **ÜBERGANGSMETALLKOMPLEXE MIT PROTONENSCHWÄMMEN ALS LIGANDEN**
TRANSITION METAL COMPLEXES WITH PROTON SPONGES AS LIGANDS
COMPLEXES DE METAL DE TRANSITION AVEC DES EPONGES A PROTONS COMME LIGANDS

(30) Priorität: 25.01.2001 DE 10103244
(43) Veröffentlichungstag der Anmeldung: 22.10.2003
(73) Patentinhaber: Studiengesellschaft Kohle mbH, 45470 Mülheim (DE)
(72) Erfinder: WÜSTEFELD, Hans-Ulrich, 46236 Bottrop (DE); KASKA, William, C., Goleta, CA 93117 (US); STUCKY, Galen, D., Goleta, CA 93117 (US); SCHÜTH, Ferdi, 45468 Mülheim/Ruhr (DE); KREBS, Bernt, 48163 Münster (DE)
(86) Internationale Anmeldenummer: PCT/EP2002/000494
(87) Internationale Veröffentlichungsnummer: WO 2002/059134

(56) Entgegenhaltungen:
- EP-A- 0 136 012
- PERIANA, ROY A. ET AL: "Platinum catalysts for the high-yield oxidation of methane to a methanol derivative" SCIENCE (WASHINGTON, D. C.) , Bd. 280, Nr. 5363, 1998, Seiten 560-564, XP002199989 in der Anmeldung erwähnt
- HARTWIG, JOHN F.: "Transition metal catalyzed synthesis of arylamines and aryl ethers from aryl halides and triflates: scope and mechanism" ANGEWANDTE CHEMIE, INTERNATIONAL EDITION, Bd. 37, Nr. 15, 1998, Seiten 2046-2067, XP002199990 in der Anmeldung erwähnt
- BELETSKAYA, IRINA P. ET AL: "The Heck Reaction as a Sharpening Stone of Palladium Catalysis" CHEMICAL REVIEWS (WASHINGTON, D. C.), Bd. 100, Nr. 8, 2000, Seiten 3009-3066, XP002199991 in der Anmeldung erwähnt

## Beschreibung

Die vorliegende Erfindung betrifft neue Übergangsmetallkomplexe mit Chino[7,8-h]chinolin oder Derivaten davon (I) sowie Cyclopentadieno[1,2-h : 4,3-h']dichinolin oder Derivaten davon (II) als Liganden, ein Verfahren zu ihrer Herstellung und ihre Verwendung als Katalysatoren für Heck-, Aminierungs- und C-H- Aktivierungsreaktionen und als Cytostatika.

Die Liganden I und II mit den Substituenten R = H und Y = H sind als Protonenschwämme bekannt (M. A. Zirnstein, Dissertation, Universität Heidelberg, 1989). Protonenschwämme sind gekennzeichnet durch geringen Stickstoff-Stickstoff-Abstand, hohe Basizität und recht niedrige Protonierungsgeschwindigkeit.

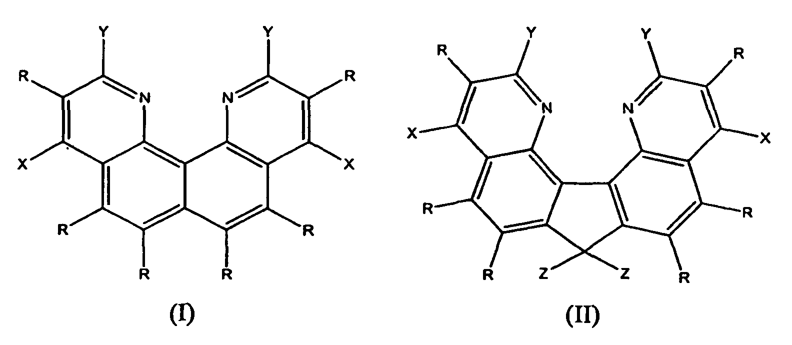

Bisher dienten Protonenschwämme, wie das 1968 von R. W. Alder synthetisierte 1,8-Bis(dimethylamino)naphthalin (R.W. Alder, P.S. Bowman, W.R.S. Steele und D.R. Winterman, J. Chem. Soc. Chem. Comm. **1968**, 723) und Chino[7,8-h]chinolin (M. A. Zimstein, H. A. Staab, Angew. Chem. **1987**, 99, 460) als Hilfsbasen für organische Reaktionen. Übergangsmetallkomplexe mit Protonenschwämmen als Liganden waren bisher nicht bekannt.

Es ist uns nunmehr gelungen, die erfindungsgemäßen Übergangsmetallkomplexe III und IV durch Reaktion eines Precursorkomplexes mit den Liganden I bzw. II darzustellen, wobei der Precursorkomplex das Übergangsmetall M und die an M gebundenen Substituenten L enthält und mindestens 2 Koordinationsstellen von M durch schwach koordinierende Liganden L besetzt sind, z. B. einem Lösungsmittel, einer CO-Gruppe oder einem π-System, z. B. Ethen. Die Darstellung der Liganden I bzw. II kann in bekannter Weise erfolgen (DE 38 14 213 A1).

Die Reaktion erfolgt in Anwesenheit eines aprotischen Lösungsmittels, z. B. halogenierte Kohlenwasserstoffe, insbesondere Dichlormethan oder Chloroform, oder von THF oder einem anderen chemisch stabilen Ether.

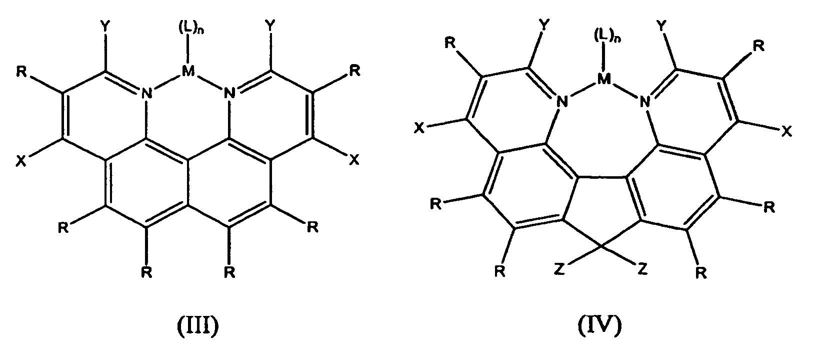

In I, II, III und IV bedeuten: X = Wasserstoff, Halogen, Alkoxy-, OH-, Nitro- oder Aminogruppe, wobei die beiden X-Substituenten gleich oder verschieden sein können; Y = Wasserstoff, Halogen, Carboxyl-, Carboxylat-, Alkyl- oder funktionalisierte Alkylgruppe OH- oder Aminogruppe, wobei die beiden Y-Substituenten gleich oder verschieden sein können und zwei R-Substituenten gemeinsam Bestandteil eines Ringsystems sein können; L = beliebiger Substituent, bevorzugt Halogen, Alkyl-, Carbonyl- oder Carboxylatgruppe; R = beliebiger Substituent, vorzugsweise Wasserstoff, Halogen, Alkyl und Derivate, Aryl und Derivate, Sulfonsäure-Gruppe, Carboxylat-Gruppe und Amino-Gruppe, wobei die Substituenten R gleich oder verschieden sein können und zwei R-Substituenten gemeinsam Bestandteil eines Ringsystems sein können; Z = Wasserstoff, Alkyl- oder Arylgruppe, wobei die beiden Substituenten Z gleich oder verschieden sein können, oder die zwei Substituenten zusammen =O sind und n = 0 bis 6 ist. Als Übergangsmetall M kommt ein Metall der Gruppen 3, 4, 5, 6, 7, 8, 9, 10, 11 oder 12 des Periodensystems in Frage, wobei die entstehenden Komplexe jedoch unterschiedlich stabil sind. Komplexe mit einem Metall der Gruppen 7, 8, 9, 10, 11 oder 12 sind stabiler als andere.

Die erfindungsgemäßen Komplexe stellen die ersten stabilen Übergangsmetallkomplexe mit einem Protonenschwamm als Liganden dar.

Die Bildung von Metallkomplexen der Formel III ist überraschend, weil der äußerst geringe N-N-Abstand in den Liganden I (beim 4,9-Dichlorchino[7,8-h]chinolin: 2.69 Å) und II und die damit verbundene sterische Hinderung dies nicht vermuten ließen. Die Komplexe zeichnen sich durch eine "out-of-plane"-Position des Metallatoms sowie extrem hohe thermische und chemische Stabilität aus. "Out-of-plane"-Position bedeutet in diesem Fall, dass das komplexierte Metallatom sich außerhalb der durch das aromatische System definierten, besten Ebene befindet. Mit bester Ebene ist die Ebene gemeint, die das kleinste Abweichungsquadrat bezüglich der Abstände aller Kohlenstoff- und Stickstoffatome des Liganden besitzt. Das Platinatom von Verbindung V befindet sich beispielsweise in einem Abstand von 1.43 Å und das Rheniumatom von Verbindung VII von 1.42 Å zu der so definierten Ebene. Diese hinsichtlich des Abstandes zur definierten Ebene außergewöhnliche Position des Metallatoms ist bisher einzigartig.

Die Stabilität der Komplexe ebenso wie die Labilität der Liganden der Metallkomplexe ist bei diesen Systemen durch Variation der X-Substituenten über einen weiten Bereich einstellbar, da der pK-Wert der Protonenschwamm-Liganden so um bis zu 6 Einheiten verändert werden kann. Bei ungleicher Substitution der beiden X-Substituenten ist chirale Katalyse möglich.
Durch sterisch anspruchsvolle Y- Substituenten kann beispielsweise das Metallatom abgeschirmt werden. Außerdem wird bei ungleicher Substitution durch sterisch anspruchsvolle Substituenten auf der einen, sterisch weniger anspruchsvolle Substituenten auf der anderen Seite chirale Katalyse möglich.

Die Synthese eines Metallkomplexes der Protonenschwämme I und II stellte eine große Herausforderung dar, weil die Precursor-Komplexe und das Lösungsmittel wegen der hohen Basizität der Liganden und deren geringem N-N-Abstand einige Voraussetzungen erfüllen müssen.

Vorteilhafte Eigenschaften potentieller Precursor-Komplexe:
- Ein Teil der Liganden des Precuror-Komplexes sollte labil und thermodynamisch nicht stark gebunden sein, so daß diese leicht substituiert werden können.
- Der Precursor-Komplex sollte im basischen Milieu zumindest für einige Stunden stabil sein.
- Idealerweise besetzt ein schwach komplexierendes Lösungsmittel eine Koordinationsstelle des Metallatoms.

Als Precursor-Komplex kann beispielsweise ein Übergangsmetallcarbonylhalogenid eingesetzt werden, wie Di-µ-chloro-dichlorobisethylenplatin (II), cis-Dichlor(1,5-cyclooctadien)-palladium (II), als Dimer vorliegendes Rhenium(I)-tetracarbonylbromid oder Mangan(1)-pentacarbonylbromid.

Vorteilhafte Eigenschaften des Lösungsmittels:
- Das Lösungsmittel sollte nicht protisch sein, da der Protonenschwamm ansonsten protoniert wird, was ihn aufgrund der starken N-H-Bindung unreaktiv für Komplexbildungsversuche macht.
- Des weiteren sollte das Lösungsmittel möglichst auch keine schwache C-H-Bindung aufweisen, dessen H-Atom der Protonenschwamm abstrahieren kann. Dadurch werden seine Koordinationsstellen zum einen blockiert, zum anderen entstehen reaktive Spezies (Carbene) mit vielfältigen Reaktionsmöglichkeiten. Produktmischungen sind die Folge.
- Das Lösungsmittel sollte in basischem Milieu möglichst stabil sein.
- Das Lösungsmittel sollte schwach an das Metallatom des Precursors koordinieren.

Beispiele für erfindungsgemäße Übergangsmetallkomplexe sind:

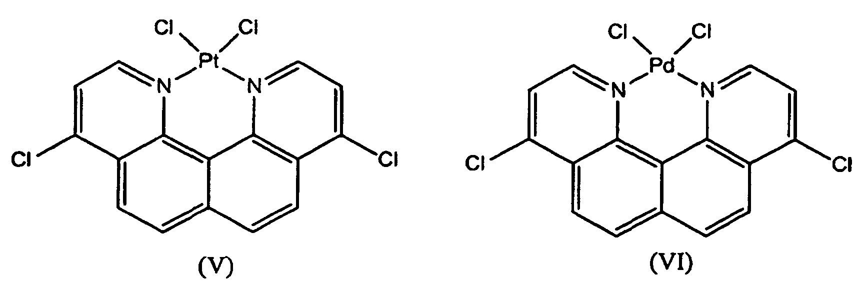

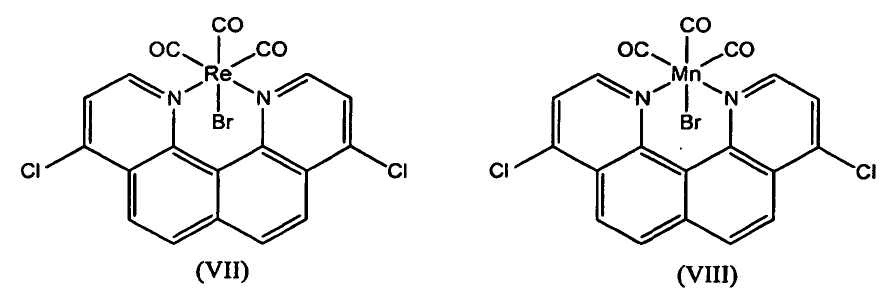

Aufgrund ihrer Stabilität und der möglicherweise zu erhöhter Reaktivität führenden "out-of-plane"-Position des Metallatoms eignen sich die erfindungsgemäßen Übergangsmetallkomplexe gut als Katalysatoren für diverse Reaktionen: Heck-Reaktion (z. B. mit Verbindung VI als Katalysator), Olefinoxidation, Polymerisation, z. B. von Ethen, sowie für Aminierungsreaktionen.

Ein weiterer Gegenstand der vorliegenden Erfindung ist demgemäß ein Verfahren in Form der "Heck-Reaktion" zur katalysierten Darstellung von olefinierten Aromaten oder Heteroaromaten, in welchem die oben dargestellten erfindungsgmäßen Übergangsmetallkomplexe mit Pd als Übergangsmetall M als Katalysatoren eingesetzt werden

Gute Heck-Katalysatoren besitzen bisher fast ausschließlich phosphanhaltige Liganden, die oxidationsempfindlich und thermisch nicht sehr stabil sind. Stickstoffhaltige Liganden bei Heck-Katalysatoren sind in Form von nicht ausreichend stabilen Komplexen bekannt, was zu geringen Ausbeuten bzw. erhöhtem Katalysatorverbrauch führt. (I. P. Beletakaya, A. V. Cheprakov, Chem. Rev. **2000**, 100, 3009).

Erfolgreiche Heck-Reaktionen mit VI als Katalysator sind mit den Beispielen 5 und 6 belegt. Der Vorteil dieses phosphanfreien Systems gegenüber gebräuchlichen phosphan-haltigen Systemen liegt in der geringen Tendenz der Stickstoffatome oxidiert zu werden und in der hohen thermischen Stabilität.

Auch für Aminierungsreaktionen kamen bislang vornehmlich phosphanhaltige Katalysatoren zum Einsatz, die jedoch ebenfalls thermisch wenig stabil sind und leicht oxidiert werden können. (J. F. Hartwig, Angew. Chem. **1998**, 110, 2154).

Noch ein weiterer Gegenstand der vorliegenden Erfindung ist ein Verfahren zur katalytischen Aminierung, in welchem die oben dargestellten erfindungsgemäßen Übergangsmetallkomplexe mit Pd oder Pt als Übergangsmetall M als Katalysator eingesetzt werden.

Die erfindungsgemäßen Katalysatorsysteme zeichnen sich dagegen durch hohe thermische und chemische Stabilität aus. Insbesondere ist es vorteilhaft, dass die Katalysatoren auch oxidationsstabil sind, da somit eine höhere "Turn Over Number" (TON) erreicht werden kann.

Dies trifft z. B. auf Palladium-Komplexe mit Chino[7,8-h]chinolin-Liganden zu, die Stickstoff-Donor-Liganden besitzen, da sie thermisch sehr stabil (bis ca. 380 °C) sind und auch nicht leicht oxidiert werden können. Sie sind in konz. Schwefelsäure stabil.

Die Stabilität der Komplexe in Schwefelsäure zeigt Anwendungspotential dieser Verbindungen als Katalysatoren für die C-H-Aktivierung, speziell die Oxidation von Methan zu Methanolderivaten. (R.A. Periana et al., Science 1998, 280, 560).

Noch ein weiterer Gegenstand der vorliegenden Erfindung ist ein Verfahren zur katalytischen C-H-Aktivierung, in welchem die oben dargestellten erfindungsgemäßen Übergangsmetallkomplexe als Katalysator eingesetzt werden. Ein Beispiel für ein derartiges Verfahren ist die Oxidation von Methan zu Methanolderivaten.

Die Platin- und Palladium-Komplexe sind erfahrungsgemäß gute, dem [cis-Diamindichloroplatin (II)] analoge Cytostatika. Durch Variation der Substituenten X am Aromaten ist es zum einen möglich, die Labilität der Chloratome an den Metallatomen über einen weiten Bereich zu steuern, zum anderen führt die "out-of-plane"-Position des Metallatomes zu erhöhter Reaktivität.

Weiterhin betrifft die vorliegende Erfindung die Verwendung der erfindungsgemäßen Übergangsmetallkomplexe III und IV zur Herstellung eines Medikaments, nämlich von Cytostatika.

Die cytostatischen Eigenschaften der Platin-Komplexe mit Protonenschwämmen sind in Anologie zu den Eigenschaften des äußerst erfolgreichen cis-Diammindichloroplatin (II) zu sehen.
In den letzten 30 Jahren konnten eine große Anzahl von Platinkomplexen synthetisiert und auf ihre cytostatische Aktivität hin untersucht werden. Durch Auswertung dieser Daten konnten empirische Struktur-Aktivitäts-Beziehungen gefunden werden (B. Lippert, Chemistry and Biochemistry of a Leading Anticancer Drug, Wiley-VCH, **1999**). Die hier beanspruchten Platinkomplexe entsprechen solchen Struktur-Aktivitäts-Beziehungen, wie viele andere Platinkomplexe auch, weisen jedoch darüber hinaus zwei bedeutende weitere Eigenschaften für Aktivität auf: Die Substitution der Gruppen in 4- und 9-Stellung des Chino[7,8-h]chinolins lässt ein Einstellen der Labilität (kinetisch) der L-Substituenten am Platin über mehrere Größenordnungen zu.
Vorteilhaft ist auch, daß die Löslichkeit dieser Verbindungen durch Substitution der Protonen am Naphthalin-Gerüst gegen polare Gruppen, z.B. SulfonsäureGruppen gut zu kontrollieren ist, ohne die Eigenschaften des Reaktionszentrums zu beeinflussen. Die Labilität der L-Substituenten am Platin lässt sich durch verschiedene Substituenten in 4- und 9-Stellung variieren, wodurch die Hydrolysegeschwindigkeit und somit die Brauchbarkeit als Cytostatikum steuerbar ist.

### Beispiele:

In den Beispielen 1 bis 4 sind Synthesevorschriften für die Übergangsmetallkomplexe V - VIII sowie deren Charakterisierung einschließlich der graphischen Darstellung der Kristallstruktur angeführt. Die Beispiele 5 - 6 zeigen Katalyse-Experimente mit VI als Katalysator.

### Beispiel 1:

### Synthese von Pt(chchCl₂)Cl₂:

### ("chchCl₂" steht für 4,9-Dichlorchino[7,8-h]chinolin)

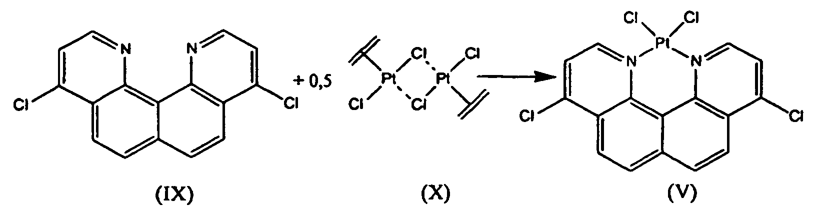

59,8 mg (0,2 mmol) 4,9-Dichlorchino[7,8-h]chinolin wurden in 20 ml getrocknetem Dichlormethan unter Argon gelöst. Diese Lösung wurde über 2 h zu einer siedenden Lösung von 58,8 mg (0,1 mmol) Di-µ-chloro-dichloro-bisethylenplatin X in Dichlormethan zugetropft und unter Rückfluß bei vollständiger Zugabe noch eine weitere Stunde auf Siedetemperatur gehalten, dann auf Raumtemperatur abgekühlt und die klare gelbe Lösung filtriert. Es wurde auf 15 ml Lösungsmittelvolumen eingeengt, und die ausfallende kräftig gelbe Substanz abgesaugt. Anschließend wurde mit je 20 ml eiskaltem Dichlormethan und Chloroform gewaschen und aus Dichlormethan umkristallisiert.

### Charakterisierung:

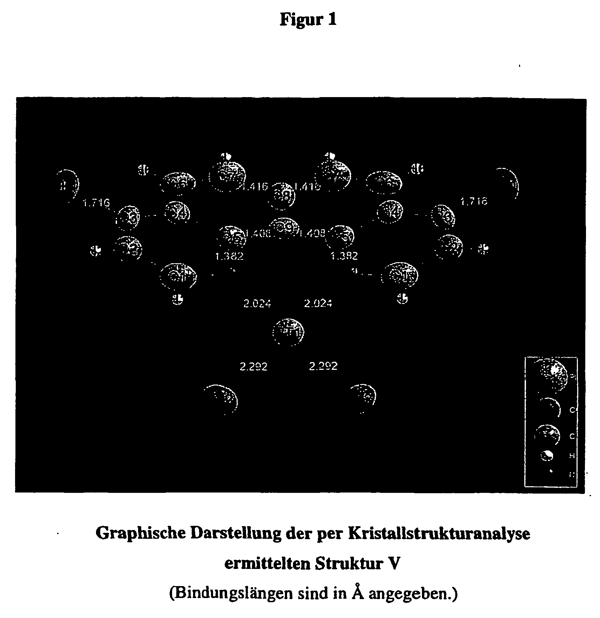

Das Ergebnis der Kristallstrukturanalyse ist in Figur 1 wiedergegeben.
Massenspektrum (EI):
m/z = 563 (4.98%, M⁺, Isotopenmuster für 4Cl), 528 (10.24%, M⁺-Cl, Isotopenmuster für 3Cl), 492 (6.18%, M⁺-HCl-Cl, Isotopenmuster für 2Cl), 456 (8.56%, M⁺-2HCl-Cl)
IR-Spektrum (KBr):
ν = 3123 (w), 3091 (m), 3060 (m), 3039 (w), 1611 (s), 1575 (s), 1559 (m), 1520 (w), 1478 (s), 1409 (s), 1348 (s), 1221 (s), 1199 (s), 1028 (s), 857 (s), 845 (s), 766 (m), 704 (s), 677 (m)

### Beispiel 2:

### Synthese von Pd(chchCl₂)Cl₂:

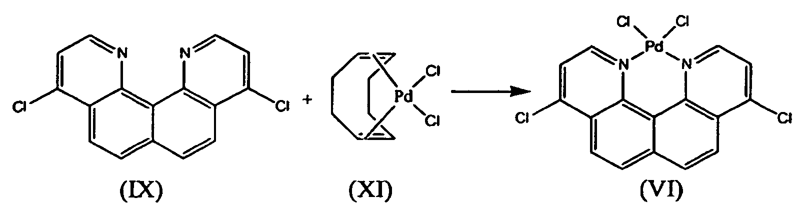

59,8 mg (0,2 mmol) 4,9-Dichlorchino[7,8-h]chinolin wurden in 30 ml einer getrockneten 8 : 1 Mischung Dichlormethan / Chloroform gelöst. Diese Lösung wurde unter Rückfluß und Argon über 1,5 h zu 20 ml einer Lösung von 57,1 mg (0,2 mmol) cis-Dichlor(1,5-cyclooctadien)palladium XI getropft. Nach beendeter Zugabe wurde noch 2 h unter Rückfluß erhitzt. Anschließend wurde auf 20 ml Lösungsmittelvolumen eingeengt und der Lösung 10 ml Dichlormethan hinzugefügt. Dann wurde nach gutem Durchmischen auf 10 ml Lösungsmittelvolumen eingeengt. Nach Abkühlung auf Raumtemperatur wurde das ausgefallene gelbe Pulver abfiltriert und mit 20 ml eiskaltem Dichlormethan gewaschen.
Massenspektrum (EI):
m/z = 476 (0.11%, M⁺), 441 (0.23%, M⁺-Cl, Isotopenmuster für Pd / 3Cl), 406 (0.19%, M⁺-2Cl, Isotopenmuster für Pd / 2Cl),
371 (0.10%, M⁺-3Cl)
IR-Spektrum (KBr):
ν = 3089 (m), 3060 (m), 2959 (m), 2931 (m), 1707 (m), 1609 (s), 1576 (m), 1516 (m), 1478 (m), 1408 (m), 1348 (m), 1253 (m), 1191 (m), 1026 (s), 855 (s), 847 (s), 768 (m), 704 (m), 678 (w), 632 (w)

### Beispiel 3:

### Synthese von Re(chchCl₂)(CO)₃Br:

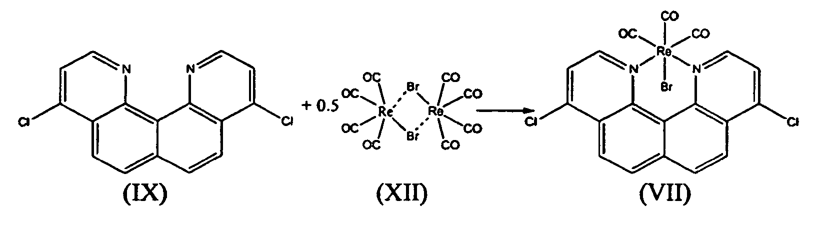

Unter Argon wurden in 50 ml THF 75,6 mg (0,1 mmol) als Dimer vorliegendes Rheniumtetracarbonylbromid und 59,8 mg (0,2 mmol) 4,9-Dichlorochino[7,8-h]chinolin suspendiert. Die Mischung wurde unter Argon 5 Tage unter Rückfluß erhitzt. Die Lösung veränderte im Laufe der ersten 2 Tage ihre Farbe von hellbraun nach orange-rot. Am dritten Tag fiel ein feinkörniger dunkeloranger Stoff aus. Nach beendeter Reaktion (5 Tage) wurde auf 10 ml eingeengt und auf Raumtemperatur abgekühlt. Das Produkt wurde unter Argon abgesaugt und mit 10 ml eiskaltem THF und Dichlormethan gewaschen. Es wurde aus 20 ml THF unter Argon umkristallisiert, abgesaugt und mit THF, Hexan und Dichlormethan gewaschen.

### Charakterisierung:

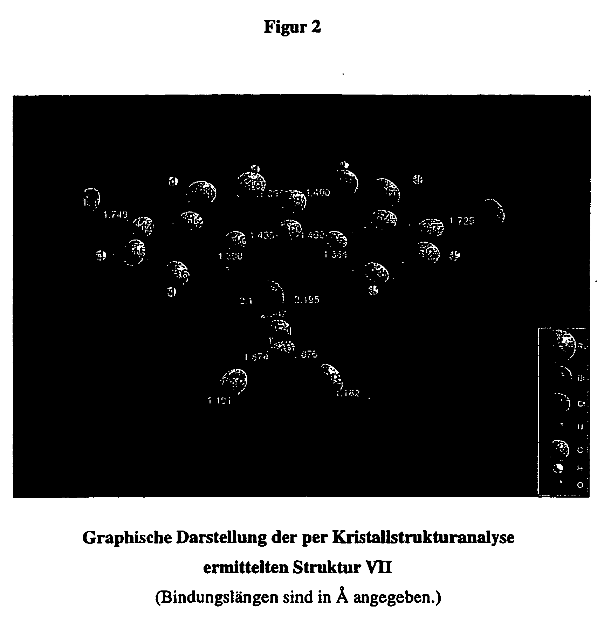

Das Ergebnis der Kristallstrukturanalyse ist in Figur 2 wiedergegeben.
Massenspektrum (EI):
m/z = 648 (51.71 %, M⁺, entspr. Isotopenmuster), 620 (31.18%, M⁺- CO, entspr. Isotopenmuster), 592 (94.95%, M⁺- 2CO, entspr. Isotopenmuster), 564 (28.06%, M⁺- 3CO)
Exakte Masse (EI) [amu]: 647.866 (ber.: 647.8655)
IR-Spektrum (KBr):
ν = 2962 (s), 2927 (s), 2854 (m), 2020 (s, ν_{C-O}), 1920 (s, ν_{C-O}), 1890 (s, ν_{C-O}), 1730 (w), 1614 (w), 1577 (w), 1553 (w), 1467 (w), 1023 (s), 864 (m), 843 (m), 701 (m)

### Beispiel 4:

### Synthese von Mn(chchCl₂)(CO)₃Br:

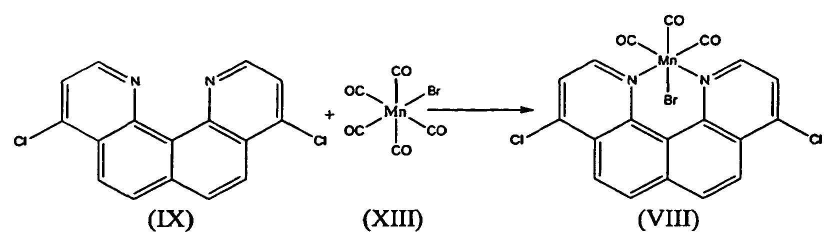

In 30 ml THF wurden 29,9 mg (0,1 mmol) 4,9-Dichlorchino[7,8-h]chinolin und 27,5 mg (0,1 mmol) Mangan(I)-pentacarbonylbromid unter Argon suspendiert. Die Lösung wurde 7 Tage unter Rückfluß erhitzt. Es fiel ein weiß-gräuliches Pulver aus, das wie Verbindung VI aufgearbeitet wurde.
Massenspektrum (EI): m/z = 432 (0.61 %, M⁺ - 3CO)
IR-Spektrum (KBr):
ν = 3064 (w), 2963 (m), 2022 (m, ν_{C-O}), 1933 (m, ν_{C-O}), 1914 (m, ν_{C-O}), 1608 (s), 1579 (s), 1550 (s), 1509 (s), 1480 (s), 1407 (s), 1021 (s), 849 (m), 839 (m), 769 (m), 695 (m) 683 (w), 633 (w)

### Beispiel 5:

### Heck-Reaktion und Ausbeuten mit VI als Katalysator sowie zum Vergleich mit dem Standard-Heck-Katalysator Palladiumacetat / Triphenylphosphan durchgeführte Reaktion:

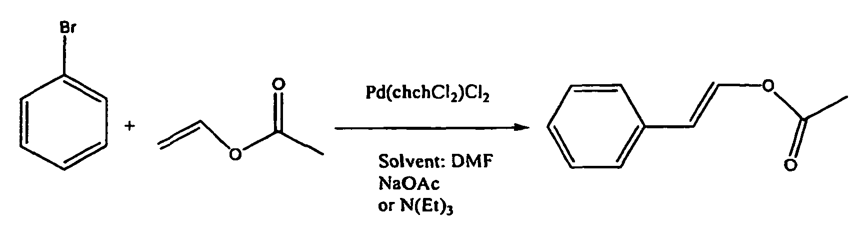

| Reaktionsbedingungen: | | | |
|---|---|---|---|
| **Katalysatorsystem** | **Ausbeute trans- Produkt [%]** | **Ausbeute cis- Produkt [%]** | **Gesamtausbeute [%]** |
| Pd(chchCl₂)Cl₂ Natriumacetat | 52.1 | 3.6 | 55.7 |
| Palladiumacetat / 4 PPh₃ Natriumacetat | 11.8 | 0.6 | 12.4 |

### Beispiel 6:

### Heck-Reaktion und Ausbeuten mit VI als Katalysator sowie zum Vergleich mit dem Standard-Heck-Katalysator Palladiumacetat / Triphenylphosphan. durchgeführte Reaktion:

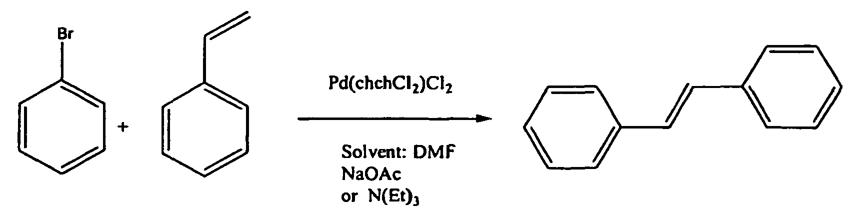

| Reaktionsbedingungen: | | | |
|---|---|---|---|
| **Katalysatorsystem** | **Ausbeute trans- Produkt [%]** | **Ausbeute cis- Produkt [%]** | **Gesamtausbeute [%]** |
| Pd(chchCl₂)Cl₂ Natriumacetat | 62.1 | 3.3 | 65.4 |
| Palladiumacetat /4 PPh₃ Natriumacetat | 11.1 | 2.1 | 13.2 |
| Pd(chchCl₂)Cl₂ Triethylamin | 37.8 | 4.3 | 42.1 |
| Palladiumacetat / 4 PPh₃ Triethylamin | 22.1 | 4.4 | 26.5 |

## Patentansprüche

1. Ein Übergangsmetallkomplex der Formeln III oder IV, 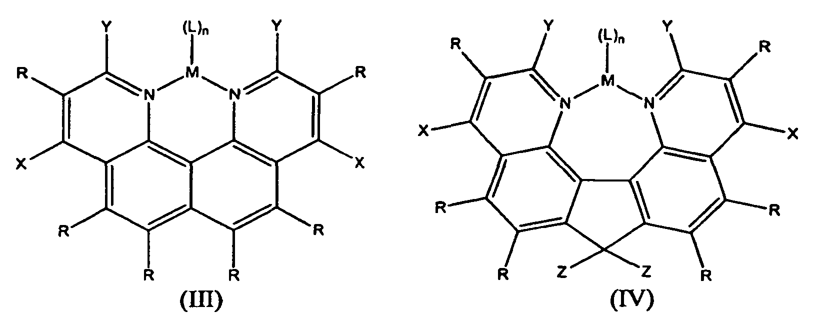 wobei
X = Halogen, Wasserstoff, Alkoxy-, OH-, Nitro- oder Aminogruppe, wobei die beiden X-Substituenten gleich oder verschieden sind;
Y = Wasserstoff, Carboxyl-, Carboxylat-, Alkyl- oder funktionalisierte Alkylgruppe, OH- oder Aminogruppe, wobei die beiden Y-Substitutenten gleich oder verschieden sind;
L = beliebiger Ligand, wobei die L-Substituenten gleich oder verschieden sind;
M =Metall, ausgewählt aus den Gruppen 3, 4, 5, 6, 7, 8, 9, 10. 11 oder 12 des PSE;
Z = Wasserstoff, Alkyl- oder Arylgruppe, wobei die beiden Z-Substituenten gleich oder verschieden oder die zwei Substituenten zusammen = O sind;
R = beliebiger Substituent, vorzugsweise Wasserstoff, Halogen, Alkyl und Derivate, Aryl und Derivate, Sulfonsäure-Gruppe, Carboxylat-Gruppe und Amino-Gruppe, wobei die Substituenten R gleich oder verschieden sind und zwei R-Substituenten gemeinsam Bestandteil eines Ringsystems sein können;
n = 0 bis 6.

2. Ein Übergangsmetallkomplex nach Anspruch 1, wobei M ein Metall ausgewählt aus den Gruppen 7, 8, 9, 10, 11 oder 12 des PSE ist.

3. Ein Übergangsmetallkomplex nach Anspruch 1, wobei L = Halogen, Alkyl-, Carbonyl-, oder Carboxylatgruppe ist.

4. Ein Übergangsmetallkomplex nach Anspruch 1 mit einer der Formeln V, VI, VII oder VIII.

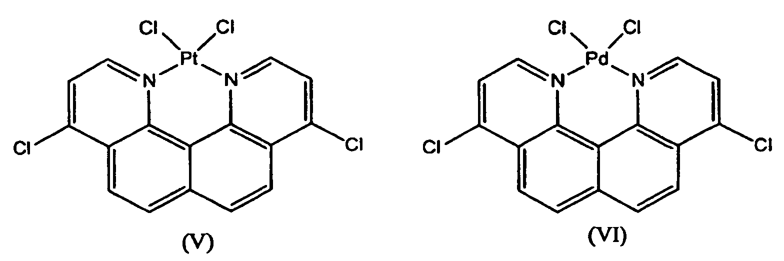

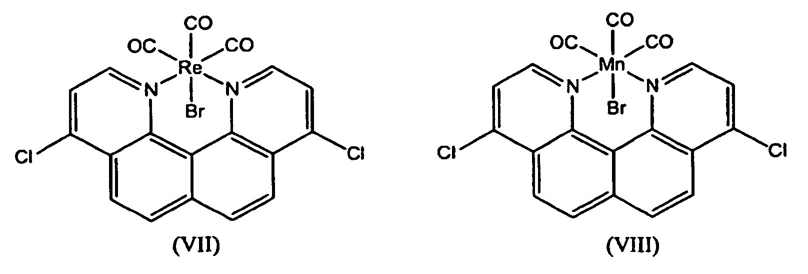

5. Verfahren zur Herstellung eines Übergangsmetallkomplexes des Anspruchs 1 durch Reaktion der Liganden I oder II mit einem Precursor-Komplex in Anwesenheit eines Lösungsmittels, wobei ein Precursor-Komplex eingesetzt wird, der das Übergangsmetall M und die an M gebundenen Substituenten L enthält und in dem mindestens zwei Koordinationsstellen von M durch schwach koordinierende Liganden besetzt sind,
wobei
X = Halogen, Wasserstoff, Alkoxy- oder Aminogruppe, Nitro- oder OH-Gruppe, wobei die beiden X-Substituenten gleich oder verschieden sind;
Y = Wasserstoff, Carboxyl-, Carboxylat-, Alkyl- oder funktionalisierte Alkyl-Gruppe, OH-Gruppe, oder Aminogruppe, wobei die beiden Y- Substitutenten gleich oder verschieden sind;
L = beliebiger Ligand, oder Halogen, Alkyl-, Carbonyl-, oder Carboxylatgruppe, wobei die L-Substituenten gleich oder verschieden sind;
M = Metall, ausgewählt aus den Gruppen 3, 4, 5, 6, 7, 8, 9, 10. 11 oder 12 des PSE;
Z = Wasserstoff, Alkyl- oder Arylgruppe, wobei die beiden Z-Substituenten gleich oder verschieden sind oder beide Z-Substituenten zusammen =O sind;
R = beliebiger Substituent, vorzugsweise Wasserstoff, Halogen, Alkyl und Derivate, Aryl und Derivate, Sulfonsäure-Gruppe, Carboxylat-Gruppe und Amino-Gruppe, wobei die Substituenten R gleich oder verschieden sind und zwei R-Substituenten gemeinsam Bestandteil eines Ringsystems sein können;
n = 0 bis 6.

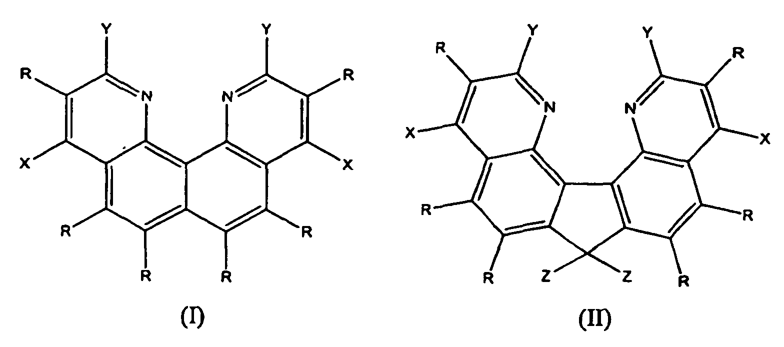

6. Verfahren nach Anspruch 5, wobei als schwach koordinierende Liganden das Lösungsmittel, eine CO-Gruppe oder ein π-System verwendet wird.

7. Verfahren nach Anspruch 5, wobei als Lösungsmittel halogenierte Kohlenwasserstoffe oder THF eingesetzt werden.

8. Verfahren nach Anspruch 7, wobei als Lösungsmittel Dichlormethan oder Chloroform eingesetzt werden.

9. Verfahren nach Anspruch 5, wobei als Precursorkomplex ein Übergangsmetallcarbonylhalogenid eingesetzt wird.

10. Verfahren nach Anspruch 9, wobei als Precursorkomplex Di-µ-chloro-dichlorobisethylenplatin (II), cis-Dichlor(1,5-cyclooctadien)-palladium (II), als Dimer vorliegendes Rhenium(I)-tetracarbonylbromid oder Mangan(I)-pentacarbonylbromid eingesetzt wird.

11. Verfahren in Form der "Heck-Reaktion" zur katalysierten Darstellung von olefinierten Aromaten oder Heteroaromaten, **dadurch gekennzeichnet, dass** als Katalysatoren Übergangsmetallkomplexe des Anspruchs 1 mit Pd als Übergangsmetall M eingesetzt werden.

12. Verfahren zur katalytischen Aminierung, **dadurch gekennzeichnet, dass** als Katalysator ein Übergangsmetallkomplex des Anspruchs 1 mit Pd oder Pt als Übergangsmetall M eingesetzt werden.

13. Verfahren zur katalytischen C-H-Aktivierung, **dadurch gekennzeichnet, dass** als Katalysator ein Übergangsmetallkomplex des Anspruchs 1 eingesetzt wird.

14. Verfahren nach Anspruch 13, wobei durch Oxidation von Methan Methanolderivate hergestellt werden.

15. Verwendung von Übergangsmetallkomplexen des Anspruchs 1 zur Herstellung eines Medikaments, nämlich eines Cytostatikums.

16. Verwendung der Übergangsmetallkomplexe nach Anspruch 15, wobei als Übergangsmetallkomplexe Platinkomplexe der Liganden I und II als Cytostatika in Analogie zum cis-Diammindichloroplatin (II) eingesetzt werden.

17. Verwendung nach Anspruch 15, wobei die Wirksamkeit oder Selektivität als Cytostatikum durch Auswahl der X-Substituenten im Komplex III oder IV eingestellt wird.

## Claims

1. A transition metal complex of the formulae III or IV, 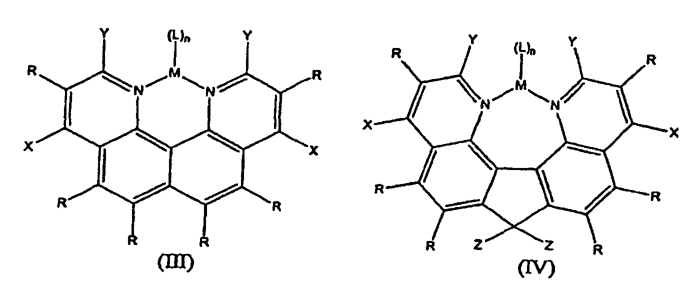 where
X = halogen, hydrogen, alkoxy, OH, nitro or amino group, where the two X substituents are identical or different;
Y = hydrogen, carboxyl, carboxylate, alkyl or functionalized alkyl group, OH or amino group, where the two Y substituents are identical or different;
L = any ligand, where the L substituents are identical or different;
M = metal selected from Groups 3, 4, 5, 6, 7, 8, 9, 10, 11 or 12 of the PTE;
Z = hydrogen, alkyl or aryl group, where the two Z substituents are identical or different or the two substituents together are O;
R = any substituent, preferably hydrogen, halogen, alkyl and derivatives, aryl and derivatives, sulphonic acid group, carboxylate group and amino group, where the R substituents are identical or different, and two R substituents may together be part of a ring system;
n = 0 to 6.

2. A transition metal complex according to Claim 1, where M is a metal selected from Groups 7, 8, 9, 10, 11 or 12 of the PTE.

3. A transition metal complex according to Claim 1, where L is halogen, alkyl, carbonyl or carboxylate group.

4. A transition metal complex according to Claim 1 having one of the formulae V, VI, VII or VIII.

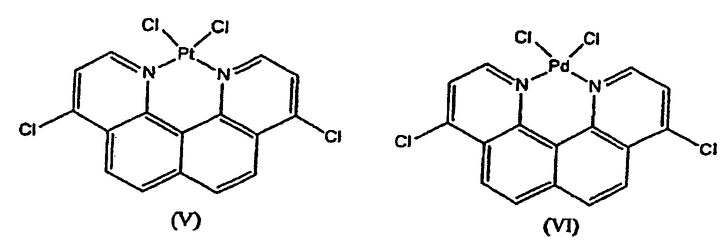

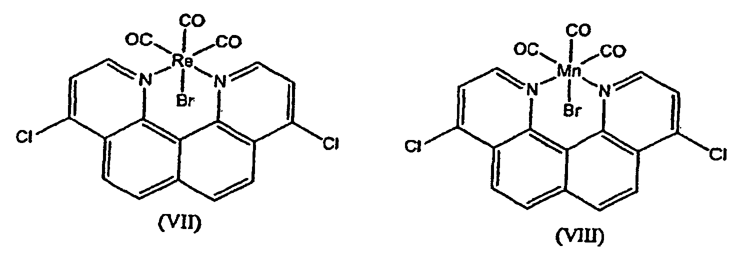

5. Process for preparing a transition metal complex of Claim 1 by reacting ligands I or II with a precursor complex in the presence of a solvent, where a precursor complex which comprises the transition metal M and the substituents L bonded to M, and in which at least two coordination sites of M are occupied by weakly coordinating ligands, is employed,
where
X = halogen, hydrogen, alkoxy or amino group, nitro or OH group, where the two X substituents are identical or different;
Y = hydrogen, carboxyl, carboxylate, alkyl or functionalized alkyl group, OH group or amino group, where the two Y substituents are identical or different;
L = any ligand, or halogen, alkyl, carbonyl or carboxylate group, where the L substituents are identical or different;
M = metal selected from Groups 3, 4, 5, 6, 7, 8, 9, 10, 11 or 12 of the PTE;
Z = hydrogen, alkyl or aryl group, where the two Z substituents are identical or different or the two substituents together are O;
R = any substituent, preferably hydrogen, halogen, alkyl and derivatives, aryl and derivatives, sulphonic acid group, carboxylate group and amino group, where the R substituents are identical or different, and two R substituents may together be part of a ring system;
n = 0 to 6.

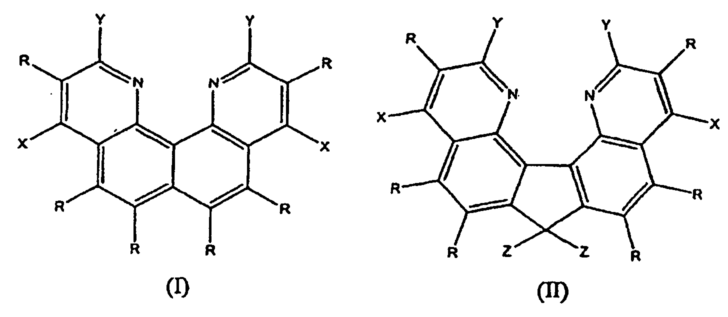

6. Process according to Claim 5, where the solvent, a CO group or a π system is used as weakly coordinating ligands.

7. Process according to Claim 5, where halogenated hydrocarbons or THF are employed as solvents.

8. Process according to Claim 7, where dichloromethane or chloroform are employed as solvents.

9. Process according to Claim 5, where a transition metal carbonyl halide is employed as precursor complex.

10. Process according to Claim 9, where di-µ-chloro-dichlorobisethyleneplatinum(II), cis-dichloro(1,5-cyclooctadiene)palladium(II), dimeric rhenium(I) tetracarbonyl bromide or manganese(I) penta-carbonyl bromide is employed as precursor complex.

11. Process in the form of the "Heck reaction" for the catalysed preparation of olefinic aromatic or heteroaromatic compounds, **characterized in that** transition metal complexes of Claim 1 with Pd as transition metal M are employed as catalysts.

12. Process for catalytic amination, **characterized in that** a transition metal complex of Claim 1 with Pd or Pt as transition metal M are employed as catalyst.

13. Process for catalytic C-H activation, **characterized in that** a transition metal complex of Claim 1 is employed as catalyst.

14. Process according to Claim 13, where methanol derivatives are prepared by oxidation of methane.

15. Use of transition metal complexes of Claim 1 for preparing a medicament, namely a cytostatic.

16. Use of the transition metal complexes according to Claim 15, where platinum complexes of ligands I and II are employed as transition metal complexes as cytostatics in analogy to cis-diaminedichloro-platinum(II).

17. Use according to Claim 15, where the activity or selectivity as cytostatic is adjusted by selecting the X substituents in the complex III or IV.

## Revendications

1. Complexe de métal de transition de formule III ou IV, 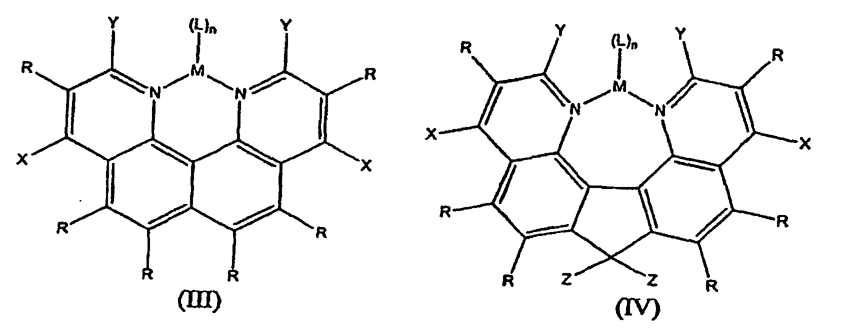 formules dans lesquelles
X représente un atome d'halogène ou d'hydrogène, un groupe alcoxy, OH, nitro ou amino, les deux substituants X étant identiques ou différents ;
Y représente un atome d'hydrogène, un groupe carboxy, carboxylate, alkyle ou alkyle fonctionnalisé, OH ou amino, les deux substituants Y étant identiques ou différents ;
L est un ligand quelconque, les substituants L étant identiques ou différents ;
M représente un métal choisi dans les groupes 3, 4, 5, 6, 7, 8, 9, 10, 11 et 12 du système périodique des éléments ;
Z représente un atome d'hydrogène, un groupe alkyle ou aryle, les deux substituants Z étant identiques ou différents, ou les deux substituants représentent ensemble O ;
R représente un substituant quelconque, de préférence un atome d'hydrogène ou d'halogène ou un groupe alkyle et dérivés, un groupe aryle et dérivés, un groupe sulfo, un groupe carboxylate ou un groupe amino, les substituants R étant identiques ou différents, et deux substituants R pouvant être ensemble un composant d'un système cyclique ;
n = 0 à 6.

2. Complexe de métal de transition selon la revendication 1, dans lequel M est un métal choisi dans les groupes 7, 8, 9, 10, 11 et 12 du système périodique des éléments.

3. Complexe de métal de transition selon la revendication 1, dans lequel L représente un atome d'halogène, un groupe alkyle, carbonyle ou carboxylate.

4. Complexe de métal de transition selon la revendication 1, ayant l'une des formules V, VI, VII et VIII.

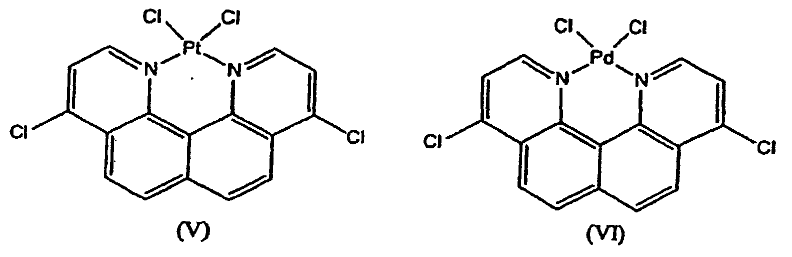

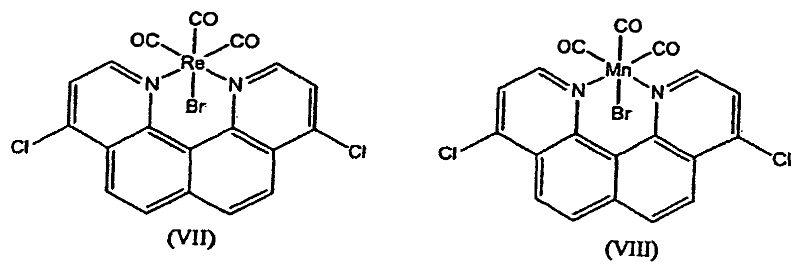

5. Procédé pour la préparation d'un complexe de métal de transition selon la revendication 1, par réaction du ligand I ou II avec un complexe précurseur en présence de solvants, dans lequel on utilise un complexe précurseur qui contient le métal de transition M et les substituants L liés à M et dans lequel au moins deux positions de coordination de M sont occupées par des ligands à faible coordination,
dans lesquels
X représente un atome d'halogène ou d'hydrogène, un groupe alcoxy ou amino, un groupe OH ou nitro, les deux substituants X étant identiques ou différents ;
Y représente un atome d'hydrogène, un groupe carboxy, carboxylate, alkyle ou alkyle fonctionnalisé, un groupe OH ou un groupe amino, les deux substituants Y étant identiques ou différents ;
L est un ligand quelconque ou un atome d'halogène, un groupe alkyle, carbonyle ou carboxylate, les substituants L étant identiques ou différents ;
M représente un métal choisi dans les groupes 3, 4, 5, 6, 7, 8, 9, 10, 11 et 12 du système périodique des éléments ;
Z représente un atome d'hydrogène, un groupe alkyle ou aryle, les deux substituants Z étant identiques ou différents, ou les deux substituants Z représentent ensemble O ;
R représente un substituant quelconque, de préférence un atome d'hydrogène ou d'halogène ou un groupe alkyle et dérivés, un groupe aryle et dérivés, un groupe sulfo, un groupe carboxylate ou un groupe amino, les substituants R étant identiques ou différents, et deux substituants R pouvant être ensemble un composant d'un système cyclique ;
n = 0 à 6.

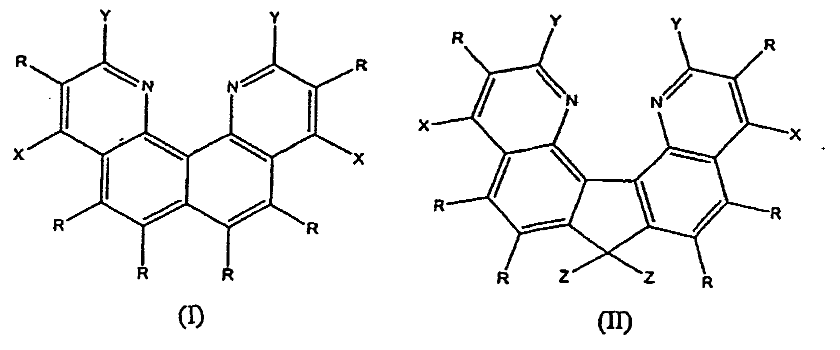

6. Procédé selon la revendication 5, dans lequel on utilise en tant que solvant à faible coordination le solvant, un groupe CO ou un système π.

7. Procédé selon la revendication 5, dans lequel on utilise en tant que solvant des hydrocarbures halogénés ou le THF.

8. Procédé selon la revendication 7, dans lequel on utilise en tant que solvant le dichlorométhane ou le chloroforme.

9. Procédé selon la revendication 5, dans lequel on utilise en tant que complexe précurseur un halogénure de carbonyl-métal de transition.

10. Procédé selon la revendication 9, dans lequel on utilise en tant que complexe précurseur le di-µ-chloro-dichlorobiséthylène-platine-(II), le *cis*-dichloro(1,5-cyclo-octadiène)palladium-(II), le bromure de pentacarbonyl-manganèse-(I) ou le bromure de tétracarbonyl-rhénium-(I) se trouvant sous forme de dimère.

11. Procédé sous forme d'une "réaction de Heck", pour la préparation catalytique de composés aromatiques ou d'hétéroatomes oléfinés, **caractérisée en ce qu'**on utilise en tant que catalyseurs des complexes de métaux de transition selon la revendication 1, comportant du Pd en tant que métal de transition M.

12. Procédé pour l'amination catalytique, **caractérisé en ce qu'**on utilise en tant que catalyseur un complexe de métal de transition selon la revendication 1, comportant du Pd ou du Pt en tant que métal de transition M.

13. Procédé pour l'activation de C-H, **caractérisé en ce qu'**on utilise en tant que catalyseur un complexe de métal de transition selon la revendication 1.

14. Procédé selon la revendication 13, dans lequel des dérivés de méthanol sont préparés par oxydation du méthane.

15. Utilisation de complexes de métaux de transition selon la revendication 1, pour la fabrication d'un médicament, à savoir d'un agent cytostatique.

16. Utilisation des complexes de métaux de transition selon la revendication 15, dans laquelle on utilise en tant que complexes de métaux de transition des complexes de platine des ligands I et II, comme agents cytostatiques par analogie avec le *cis*-diamminedichloroplatine-(II).

17. Utilisation selon la revendication 15, dans laquelle on règle l'efficacité ou la sélectivité en tant qu'agent cytostatique par le choix des substituants X dans le complexe III ou IV.
